# EUROPEAN PATENT SPECIFICATION

(11) **EP 3 765 440 B1**
(45) Date of publication and mention of the grant of the patent: **19.07.2023**
(21) Application number: 19709964.1
(22) Date of filing: 14.03.2019
(51) Int. Cl.: C07C 241/00, C07C 243/02

(54) **PROCESS FOR THE PREPARATION OF N-ALKYL-NITRATOETHYLNITRAMINES**
VERFAHREN ZUR HERSTELLUNG VON N-ALKYL-NITRATOETHYLNITRAMINEN
PROCÉDÉ DE PRÉPARATION DE LA N-ALKYL-NITRATOETHYLNITRAMINE

(30) Priority: 15.03.2018 EP 18162059
(43) Date of publication of application: 20.01.2021
(73) Proprietor: Nitrochemie Aschau GmbH, 84544 Aschau am Inn (DE)
(72) Inventor: NIEDER, Anian, 84453 Muehldorf am Inn (DE); WUENSCHE, Tobias, 84453 Muehldorf am Inn (DE); FRIEDRICHS, Anne Theresa, 81241 Munich (DE); KAINZ, Johannes, 84453 Muehldorf am Inn (DE); HUBER, Alexander, 83109 Grosskarolinenfeld (DE)
(74) Representative: Paustian, Othmar
(86) International application number: PCT/EP2019/056379
(87) International publication number: WO 2019/175296

(56) References cited:
- EP-B1- 1 196 374
- RAO KALA P C ET AL: "Studies on n-Butyl nitroxyethylnitramine (n-BuNENA): Synthesis, characterization and propellant evaluations", PROPELLANTS, EXPLOSIVES, PYROTECHNICS, WILEY - V C H VERLAG GMBH & CO., WEINHEIM, DE, vol. 29, no. 2, 1 April 2004 (2004-04-01), pages 93-98, XP002449901, ISSN: 0721-3115, DOI: 10.1002/PREP.200400035

## Description

The present invention relates to a process for the preparation of N-alkyl-nitratoethylnitramines (NENA compounds) and dioxyethylnitramine dinitrate (DINA).

Nitratoethylnitramines (NENA compounds, DINA) are of great interest as energetic plasticisers and as constituents of propellants and explosives. Important examples of nitratoethylnitramines are methyl-NENA, ethyl-NENA, butyl-NENA and DINA. The preparation of such compounds by a continuous process is described in EP 1 196 374 and in US 6,262,301. That process does have some disadvantages, however. For example, it requires a complex mechanical structure (for example as a result of the stirring device, heat exchanger, tank, lines, *etc*.), which is associated with generally high safety requirements, high maintenance and cleaning requirements and the associated longer downtimes for maintenance and cleaning, immobility of the apparatus and parts of the apparatus, as well as a large space requirement for the total apparatus. The described process also requires laborious and complicated temperature and reaction control. Furthermore, the start-up and shut-down procedure of the described process is complicated and laborious. In addition, the described process requires the initial presence of the reactants in the reactor vessels and, furthermore, large amounts of chemicals are used, resulting in a large reactive mass. Moreover, in the case of the known process only the synthesis of individual components and not of product mixtures is described.

When carrying out the process described in EP 1 196 374 at ambient pressure, a high risk of explosion was observed. When investigating the cause of the explosion, it was surprisingly found that the high ignition risk results from the formation of inflammable vapour/gas mixtures above the level of the fluid in the reactor vessel (potentially hypergolic mixture).

The problem of the present invention was accordingly to provide an improved process for the preparation of N-alkyl-nitratoethylnitramines (e.g. NENA compounds, DINA), especially to provide a process having a reduced risk of explosion.

The present invention relates to a process for the preparation of a compound of the formula (I): 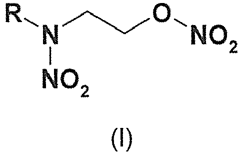
wherein R is an alkyl group having from 1 to 6 carbon atoms or a group of the formula -CH₂-CH₂-O-NO₂, or of mixtures of two or more such compounds, which comprises the reaction of a compound of the formula (II): 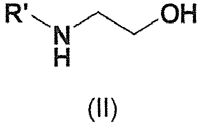
wherein R' is an alkyl group having from 1 to 6 carbon atoms or a group of the formula -CH₂-CH₂-OH, or the reaction of corresponding mixtures of two or more such compounds,
with nitric acid and then with a mixture of at least one acid anhydride and a chloride-containing catalyst in a continuous process, characterised in that the reactions are carried out at a pressure of from 2 bar above atmospheric pressure up to a pressure of 80 bar above atmospheric pressure.

In the reactions according to the invention, preference is given to a pressure of from 2 bar above atmospheric pressure up to 18 bar above atmospheric pressure. Furthermore, preference is given to a pressure of from 2 bar above atmospheric pressure up to 16 bar above atmospheric pressure. Preference is further given to a pressure of from 2 to 15 bar above atmospheric pressure. Special preference is given to a pressure of from 3 to 12 bar above atmospheric pressure; especially from 4 to 10 bar above atmospheric pressure. Elevated pressure with respect to atmospheric pressure suppresses the formation of gas bubbles and problems associated therewith.

Especially preferably, in the process of the present invention the pressure is selected such that the formation of a gas phase within the reactor is prevented during the reactions. Further preferably, in the process of the present invention the pressure is selected such that it is below the burst pressure of the reactors used.

The problems associated with the formation of gas bubbles can e.g. result from flammable bubbles of gas and vapors that pose a high risk. The bubbles may result from the reactants themselves (e.g. the organic amines used), from possible decomposition products during the reaction (e.g. nitrogen oxides such as nitrogen dioxide NO₂ from nitric acid), from overheating of the reaction mixture or from dissolved gases (e.g. air), as well as other volatiles (e.g. solvents in the raw materials), which are dissolved in the reactants, but may also be caused by mechanical movements of agitators, pumps and the like by cavitation.

By an increased pressure on the one hand, the formation of bubbles or evaporation or outgassing of the corresponding substances is suppressed, even at elevated temperatures. On the other hand, the solubility of gases and vapors in the liquid reaction medium is increased.

In the specific case of the nitrogen oxides nitrogen dioxide and dinitrogen tetroxide, which are formed in nitric acid by light/heat or by chemical action and which are dissolved therein, the pressure effectively affects the balance between NO₂ and N₂O₄. The increase in pressure therefore acts against entropy. NO₂ is a radical and thus very reactive. It is also volatile under normal conditions and tends to form ignitable mixtures (hypergol). N₂O₄, on the other hand, is less reactive and non-volatile. The same applies to the nitrogen oxides NO₂, NO and N₂O₃, as well as to N₂O₅. Since these equilibria are also thermally influenced, also a fast and efficient cooling to shift the chemical equilibrium in the direction of less problematic nitrogen oxides N₂O₄, N₂O₃ and N₂O₅ is of great safety advantage.

In the process of the present invention, both symmetrical and mixed anhydrides can be used. Examples of suitable acid anhydrides are trifluoroacetic anhydride, acetic anhydride or suitable acetyl nitrates synthesized *in situ.* Acetic anhydride is preferred.

After the reactions, the reaction mixture is preferably brought into contact with water or an aqueous alkaline solution (quenched).

It is preferred that R is a methyl, ethyl or butyl group or a group of the formula -CH₂-CH₂-O-NO₂.

It is further preferred that R' is a methyl, ethyl or butyl group or a group of the formula -CH₂-CH₂-OH.

Furthermore, the reaction with nitric acid is preferably carried out at a temperature of from -20°C to 30°C; especially at a temperature of from -10°C to 10°C or at a temperature of from -15°C to -5°C.

It is further preferred that the reaction is carried out with a mixture of at least one acid anhydride and a chloride-containing catalyst at a temperature of from 20°C to 60°C; especially at a temperature of from 27°C to 36°C or at a temperature of from 25°C to 35°C.

Examples of chloride-containing catalysts are hydrogen chloride (for example in the form of hydrochloric acid) as well as other chloride-containing catalysts, such as organic and inorganic chlorine compounds, such as chlorine salts, for example zinc(II) chloride, ammonium chloride or triethylammonium chloride, as well as semimetal chlorides, such as, for example, silicon chlorides (for example trimethylsilyl chloride and tetrachlorosilane) or titanium(IV) chloride, or organic chlorine compounds, such as, for example, different acetyl chlorides and carboxylic acid chlorides or other acid chlorides. Such chlorine compounds liberate HCl *in situ* on contact with water or the like. Typically, 0.02 - 2 mol% chloride are admixed in acetic anhydride for the reaction.

The chloride-containing catalyst used is preferably hydrogen chloride (e.g. in the form of hydrochloric acid). The use of gaseous hydrogen chloride as a chloride-containing catalyst has the advantages of preventing the entry of water, of being easy to meter and convey, and of being easy to transport and store in gas cylinders. The use of hydrogen chloride as chloride-containing catalyst especially has the advantage over the use of zinc(ll) chloride that no metal salts are formed in the reaction, which salts can contaminate or clog the reactor (especially a microreactor). Furthermore, the use of hydrogen chloride is cheaper and more environmentally friendly than, for example, the use of zinc(II) chloride. The use of hydrogen chloride also offers the advantage that it has better miscibility with the remainder of the reaction mixture and accordingly no preparation time is required for the mixing. In the process described in EP 1 196 374, for example, the zinc chloride has to be dissolved in acetic anhydride, which requires several hours.

The chloride-containing catalyst can also be introduced into the reactor already in the first reaction stage (for example with the nitric acid). Thereby, the chloride-containing catalyst may be introduced into the reactor e.g. as chlorosulfonic acid in the nitric acid, as metal chloride or complex compound (e.g. a complex of zinc chloride and one of the starting materials such as N-ethylethanolamine (EEA)) or as chloride salts of the amines, which are used as starting material (e.g. EEA * HCl).

The nitric acid used in the reaction can be used in a concentration of 65-100 % (in water). Preferably nitric acid having a concentration of from 75 to 96 % (in water) is used. Special preference is given to the use of nitric acid having a concentration of from 80 to 90 % (in water). The use of nitric acid having a concentration of from 75 to 96 % (especially from 80 to 90 %) has the advantage that the reaction can be better controlled, because the use of concentrated nitric acid involves the risk of localised overheating. It has surprisingly been found that the process according to the invention can be carried out not only with concentrated nitric acid but also with dilute nitric acid.

The process according to the invention is especially preferably used to prepare a mixture of ethyl-NENA (R = ethyl) and methyl-NENA (R = methyl); especially in a ratio by weight (ethyl-NENA : methyl-NENA) of from 60 : 40 to 30 : 70; especially preferably in a ratio by weight of from 50 : 50 to 40 : 60. The use of such a mixture offers the advantage that the presence of ethyl-NENA makes it possible to do without an additional solvent and the solid methyl-NENA does not need to be separately purified (especially by recrystallisation). Furthermore, in terms of risk during handling and processing and in respect of storage and transport guidelines, crystalline methyl-NENA is to be regarded as more critical than in a solution in ethyl-NENA.

Preferably the reactions of the present invention are carried out in one or more continuous flow reactor(s).

The use of continuous flow reactors in conjunction with the increased pressure preferably completely prevents the formation of a gaseous phase. This prevents the formation of an ignitable gas mixture and thus significantly increases the safety of the process.

Conducting reactions under pressure is rather uncommon in the synthesis of explosives, as conventional pressure reactor vessels, through their own containment, result in significant damage when ignited. Therefore, it is advantageous to carry out the process of the present invention in one (or more) continuous flow reactor(s), since the lower reaction rates would cause significantly less damage in the event of an explosion.

The continuous flow reactor used is preferably a tubular reactor.

The reactions of the present invention are especially preferably carried out in one or more tubular reactor(s).

The preferred internal volume of the tubular reactors used is from 0.1 µl to 10,000 I.

The use of continuous flow reactors (especially tubular reactors) offers the advantage of short residence times with a high throughput.

Preferably the continuous flow reactor or tubular reactor used has at least one mixer or integrated structures or chicanes which serve as mixer (especially a static mixer).

The reactions of the present invention are especially preferably carried out in one or more microreactor(s).

The use of microreactors is particularly preferred in the present invention because the narrow tube diameters prevent transmission of a deflagrating or detonative reaction beyond the explosion point.

Microreactors are, for example, miniaturised continuous flow reactors (such as, for example, tubular reactors) the smallest channel dimensions of which are, for example, in the range of from 100 to 10,000 µm. Such microreactors can e.g. be produced on the basis of metals, silicon, ceramics, polymers and glass. Known named reactions of preparative chemistry, such as *inter alia* the Wittig reaction, Knoevenagel condensation, Michael addition, Diels-Alder reaction and Suzuki coupling, have been successfully carried out in microreactors with overwhelmingly improved selectivities and conversions. Microreactors are distinguished by a very accurate and continuous procedure, a high heat exchange coefficient, very good reaction kinetics, short reactant residence time and good further processing of unstable intermediates (see, for example, US 2014/0350274 A1 and Monbaliu et al., Chem. Today 2011, 29, 50).

The use of microreactors gives rise to a number of advantages. The reactive mass and the hazardous quantities can be minimised. The reaction of small amounts with optimum temperature control (optimum surface/volume ratio and good heat transmission, for example by an integrated heat exchanger) results in excellent reaction control as well as an optimum reaction procedure (increased yields and avoidance of secondary products). Furthermore, no inflammable vapour or gas mixture is produced or accumulates (especially in the case of a reaction under pressure); any gas bubbles of reactive mixtures that do arise are flushed out. The process according to the invention is also distinguished by a simple start-up and shut-down procedure which can be readily automated ("Plug & Play"). The process of the present invention allows synthesis as required, resulting in a reduction in amounts to be stored (just-in-time production). The microreactors used require less maintenance and cleaning with the consequence of lower production downtimes in a continuous production mode than the conventional process, because no mechanical parts, such as, for example, stirring devices or heating coils, are required. The process of the present invention is resource-saving overall and the very compact structure of the microreactors requires only a small amount of space and renders the apparatus easier to move (mobile production apparatus).

The microreactors preferably used in the process of the present invention preferably comprise one or more microreactor module(s) which can be connected in parallel or in series. Such microreactor modules are preferably configured as continuous flow reactors. A microreactor can comprise a plurality of identical or different microreactor modules. The number of microreactor modules connected one after the other per microreactor can e.g. be from 1 to 15, it being preferable to use from 2 to 11 modules per microreactor.

The microreactor modules can be made from one or more materials such as glass, silicon carbide, fluorinated plastics or corrosion-resistant steels (preferably glass and/or silicon carbide).

The smallest diameter or the smallest dimension of the fluid channels (or reactant channels) of the microreactor modules is preferably from 100 µm to 10,000 µm; especially preferably from 300 µm to 7000 µm; especially from 500 µm to 3000 µm.

Preferably the total internal volume of the fluid channels (or reactant channels) of a microreactor module is from 0.5 ml to 1000 ml; more preferably from 5 ml to 500 ml; especially preferably from 10 ml to 400 ml; especially from 30 ml to 300 ml.

The length of the fluid channels (or reactant channels) of the entire microreactor is determined by the residence time, the flow rate and the internal volume of the individual microreactor modules and the reaction rate as well as the reaction temperature.

It is preferable to use microreactor modules having a temperature-control device, such as, for example, a temperature-control bath or temperature-control lines. Special preference is given to the use of reactor modules having an integrated temperature-control device. Such integrated temperature-control devices can be, for example, capillaries or layers having a temperature-control medium. Such temperature-control devices can be arranged above and/or below or around the throughflow channels in which the reactions take place. The temperature-control medium used is preferably water or water/alcohol mixtures (for example glycol, ethanol or isopropanol).

The residence times in the microreactor modules can be from a few milliseconds up to several minutes. Residence times of from 0.04 min to 1 min per microreactor module are preferred.

Suitable microreactor modules are, for example, the Corning SAS flowing modules G0, G1, G2, G3, G4. Preference is given to G0, G1, G3 and G4; special preference is given to G3 and G4. Preferred microreactor modules are described, for example, in US 8,534,909.

In the present invention, preferably a plurality of identical or different microreactors are connected one after the other in series. Especially preferably, in the present invention at least two (especially at least three) microreactors are connected in series.

It is likewise possible in the present invention to connect one or more microreactor(s) and one or more continuous flow reactors (such as, for example, tubular reactors) in series.

Preferably the first reaction between N-alkyl-ethanolamine and nitric acid is carried out in a microreactor (comprising at least one microreactor module) as a continuous process and the product from that microreactor is continuously transferred to a further microreactor (comprising at least one microreactor module), in which further microreactor acetic anhydride and the chloride-containing catalyst are added.

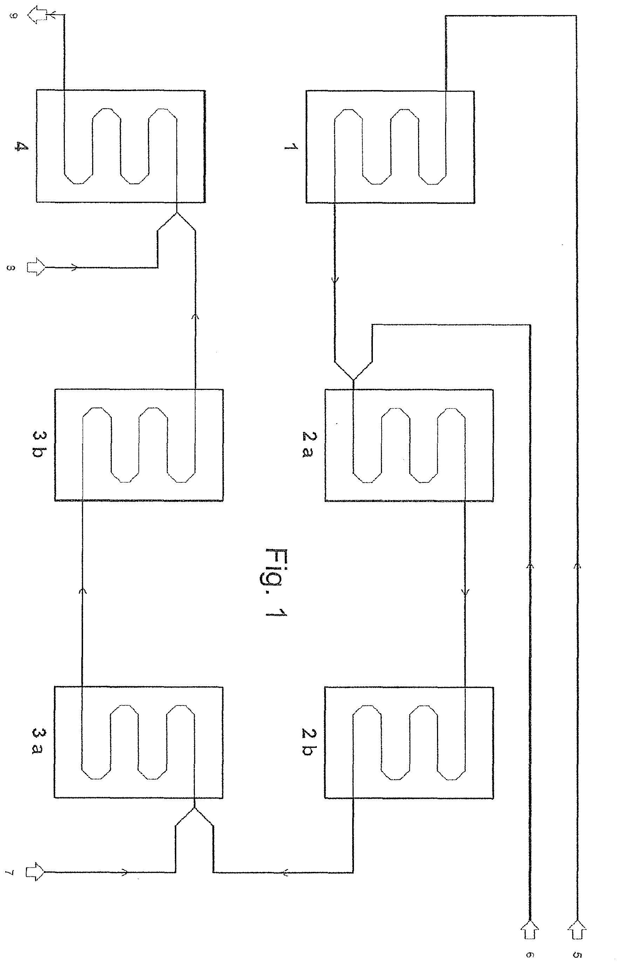

Figure 1 shows a flow diagram of a preferred device for carrying out the process according to the invention.

A preferred embodiment of the process of the present invention is described in detail below. The compound of the formula (II) or the mixture thereof is referred to as amine below.

Nitric acid (65-100 % in water, preferably 75-96 % in water, especially preferably 80-90 % in water) is pumped through a first microreactor (comprising at least one microreactor module 1) (flow rate: 10-50 ml/min, residence times of 0.1 - 1.1 min, preferably 0.4 - 0.7 min). The first microreactor serves for pre-cooling the acid to a temperature of from -20°C to 20°C (preferably from -10°C to 10°C).

In a second microreactor (comprising at least one microreactor module **2a** and optionally one or more microreactor module(s) **2b**), the acid thus cooled is brought to reaction with amine (concentration >50 % in water, preferably >96 %) with a constant flow of amine (3 - 20 g/min, preferably 5 - 15 g/min, residence time 0.1 - 0.7 min, preferably 0.2 - 0.5 min) at a temperature of from -20°C to 20°C (preferably from -10°C to 10°C).

The cooling of the nitric acid and the subsequent reaction is carried out at a pressure of from 2 bar above atmospheric pressure up to 18 bar above atmospheric pressure.

It is possible for more than one microreactor module **2a** to be arranged in the second microreactor in order to distribute the metering of amine between a plurality of microreactor modules **2a.** The metering can either be effected *via* individual metering pumps operating independently or *via* a pump and the corresponding number of metering valves.

The reaction mixture from the second microreactor is conducted into a next microreactor (comprising at least one microreactor module **3a** and optionally one or more microreactor module(s) **3b**) where it is mixed with acetic anhydride and the catalyst dissolved therein (preferred catalyst content for concentrated hydrochloric acid (>25 %): 0.5 - 5.0 % by volume, preferably 1 - 2 % by volume; flow rate 40 - 120 g/min, preferably 55 - 95 g/min; residence time 0.1 - 1.1 min, preferably 0.3 - 0.8 min) and heated to from 20°C to 40°C (preferably from 27°C to 36°C) and brought to reaction.

In a fourth microreactor (comprising at least one microreactor module **4**), the reaction solution can then be quenched with water or aqueous alkaline solution (preferably sodium hydroxide, sodium carbonate or sodium hydrogen carbonate solution in water; concentration 5 - 20 % by weight) at from 0°C to 20°C and at from 0 to 18 bar and the crude product precipitated. The advantage of this variant lies in the better temperature control as well as in the quicker and more efficient mixing and quenching.

Alternatively the reaction mixture from the third microreactor can be quenched with water and the crude product precipitated outside the fluid channels (or reactant channels) at normal pressure, the crude product being obtained preferably in a yield of from 60 to >95 mol% based on the amine and with a purity (according to HPLC) of 92 - 100 %.

In that case the reaction mixture from microreactor module **3b** is quenched with water or dilute alkaline solution (for example carbonate solution) in a mixing chamber and the reaction interrupted, the temperature preferably being controlled to 5 - 20°C.

Unlike the first variant with the microreactor module **4,** a larger amount of quenching medium can be supplied by means of a mixing chamber. It is also possible to operate at lower pressure. This improves droplet formation and facilitates outgassing of gases from the reaction or from the quenching that are bound in the solution, in this case especially when a carbonate solution is used.

The subsequent separation can be effected by means of cyclone separation, precipitation separation, membrane separation or other phase separation methods in order to separate the two resulting phases from one another.

The organic product phase from the separation can be washed by means of water or some other suitable washing medium (for example dilute alkaline solution or salt solutions) and separated by means of a second separating step (see first separation).

In addition to the adjustment of the flow rates, it is also possible for the residence time to be adjusted by modular lengthening of the fluid channels (or reactant channels) or installation of further microreactor modules.

### EXAMPLES

### Example 1: Synthesis of ethyl-NENA:

87 % nitric acid at a flow rate of 23 ml/min is cooled to -6°C at a residence time of 28 sec and conveyed into the second microreactor where, at -6°C, 11.5 g/min of N-ethylethanolamine (EEA) are metered into the pre-cooled nitric acid. After a residence time of 30 sec, acetic anhydride with 1 % by volume admixed hydrochloric acid (37 % hydrochloric acid in water) is introduced into the resulting reaction mixture at a flow of 70 g/min. The mixture is maintained at 32°C for a further 34 sec and quenched with an inflow of water of 155 g/min at 15°C. The crude product is obtained in a yield of 92 mol% (based on the amine) with a purity according to HPLC of 98 %. The pressure in the entire reactor under these test conditions is between 4.5 and 11 bar, depending upon the measurement point.

### Example 2: Synthesis of ethyl/methyl-NENA (42 % ethyl-NENA, 58 % methyl-NENA, MEN42):

85 % nitric acid at a flow rate of 30 ml/min is cooled to -6°C at a residence time of 20 sec and conveyed into the second microreactor where, at -6°C, 9.5 g/min of N-methylethanolamine/N-ethylethanolamine (MEA/EEA: 60/40 % by weight) are metered into the pre-cooled nitric acid. After a residence time of 15 sec, acetic anhydride with 1 % by weight admixed zinc(II) chloride is introduced into the resulting reaction mixture at a flow of 85 g/min. The mixture is maintained at 32°C for a further 29 sec and quenched with an inflow of water of 155 g/min at 15°C over a period of 3 sec. The crude product is obtained in a yield of 90 mol% (based on the amines) with a purity according to HPLC of 98 % and a ratio by weight of 42 % ethyl-NENA to 58 % methyl-NENA. The pressure in the entire reactor under these test conditions is between 4.5 and 12 bar, depending upon the measurement point.

### Example 3: Synthesis of ethyl/methyl-NENA (52 % ethyl-NENA, 48 % methyl-NENA, MEN52):

85 % nitric acid at a flow rate of 30 ml/min is cooled to -6°C at a residence time of 20 sec and conveyed into the second microreactor where, at -6°C, 10.0 g/min of N-methylethanolamine/N-ethylethanolamine (MEA/EEA: 50/50 % by weight) are metered into the pre-cooled nitric acid. After a residence time of 15 sec, acetic anhydride with 1 % by volume admixed hydrochloric acid (37 % hydrochloric acid in water) is introduced into the resulting reaction mixture at a flow of 80 g/min. The mixture is maintained at 32°C for a further 30 sec and quenched with an inflow of 8 % sodium hydroxide solution, 150 g/min, at 20°C for 3 sec. The crude product is obtained in a yield of 94 mol% (based on the amines) with a purity according to HPLC of 98.5 % and a ratio by weight of 52 % ethyl-NENA to 48 % methyl-NENA. The pressure in the entire reactor under these test conditions is between 4.5 and 12.5 bar, depending upon the measurement point.

Alternatively, if dilute nitric acid is used, dilution of highly concentrated nitric acid (99-100 % nitric acid) with water can be carried out upstream of the actual synthesis. This is described below:

### Example 4: Synthesis of ethyl-NENA with integrated acid pre-mixing:

In the first microreactor, 99 % nitric acid is cooled to -6°C at a flow of 26 ml/min over a period of 24 sec. The cooled acid is then diluted with 6.0 ml of water at -6°C over a period of 18 sec. The 87 % nitric acid so mixed is then conditioned at -6°C at a residence time of 19 sec and conveyed into the second microreactor where, at -6°C, 13 g/min of N-ethylethanolamine (EEA) are metered into the pre-cooled dilute nitric acid. After a residence time of 13 sec, acetic anhydride with 1 % by volume admixed hydrochloric acid (37 % hydrochloric acid in water) is introduced into the resulting reaction mixture at a flow of 80 g/min. The mixture is maintained at 33°C for a further 29 sec and quenched with an inflow of water of 155 g/min at 20°C over a period of 3 sec. The crude product is obtained in a yield of 90 mol% (based on the amine) with a purity according to HPLC of 97 %. The pressure in the entire reactor under these test conditions is between 4.5 and 12.5 bar, depending upon the measurement point.

### Reference signs

- 1: Microreactor module of the first microreactor
- 2a: Microreactor module of the second microreactor
- 2b: Microreactor module of the second microreactor
- 3a: Microreactor module of the third microreactor
- 3b: Microreactor module of the third microreactor
- 4: Microreactor module of the fourth microreactor
- 5: Supply of nitric acid
- 6: Supply of amine
- 7: Supply of acetic anhydride and chloride-containing catalyst
- 8: Supply of water or aqueous alkaline solution (quenching solution)
- 9: Working-up/separation

## Claims

1. Process for the preparation of a compound of the formula (I): 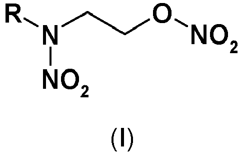
wherein R is an alkyl group having from 1 to 6 carbon atoms or a group of the formula -CH₂-CH₂-O-NO₂, or of mixtures of two or more such compounds, which comprises the reaction of a compound of the formula (II): 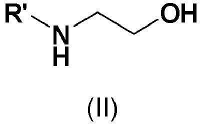
wherein R' is an alkyl group having from 1 to 6 carbon atoms or a group of the formula -CH₂-CH₂-OH, or the reaction of corresponding mixtures of two or more such compounds,
with nitric acid and
then with a mixture of at least one acid anhydride and a chloride-containing catalyst in a continuous process,
**characterised in that** the reactions are carried out at a pressure of from 2 bar above atmospheric pressure up to a pressure of 80 bar above atmospheric pressure.

2. Process according to claim 1, **characterised in that** after the reactions the reaction mixture is brought into contact with water or an aqueous alkaline solution.

3. Process according to claim 1 or 2, **characterised in that** R is a methyl, ethyl or butyl group or a group of the formula -CH₂-CH₂-O-NO₂ and R' is a methyl, ethyl or butyl group or a group of the formula -CH₂-CH₂-OH.

4. Process according to any one of the preceding claims, **characterised in that** the reactions are carried out in one or more continuous flow reactor(s).

5. Process according to any one of preceding claims 1 to 3, **characterised in that** the reactions are carried out in one or more tubular reactor(s).

6. Process according to any one of preceding claims 1 to 3, **characterised in that** the reactions are carried out in one or more microreactor(s).

7. Process according to any one of the preceding claims, **characterised in that** the reactions are carried out at a pressure of from 2 bar above atmospheric pressure up to a pressure of 18 bar above atmospheric pressure, preferably at a pressure of from 3 to 12 bar above atmospheric pressure.

8. Process according to any one of the preceding claims, **characterised in that** the reaction with nitric acid is carried out at a temperature of from -20°C to 30°C; especially at a temperature of from -10°C to 10°C or at a temperature of from -15°C to -5°C.

9. Process according to any one of the preceding claims, **characterised in that** the reaction with a mixture of at least one acid anhydride and a chloride-containing catalyst is carried out at a temperature of from 20°C to 60°C; especially at a temperature of from 27°C to 36°C or at a temperature of from 25°C to 35°C.

10. Process according to any one of the preceding claims, **characterised in that** acetic anhydride is used as acid anhydride.

11. Process according to any one of the preceding claims, **characterised in that** hydrogen chloride is used as chloride-containing catalyst.

12. Process according to any one of the preceding claims, **characterised in that** the nitric acid used in the reaction is used with a concentration of from 75 to 96 % in water.

13. Process according to any one of the preceding claims, **characterised in that** a mixture of ethyl-NENA (R = ethyl) and methyl-NENA (R = methyl) is prepared.

14. Process according to any one of the preceding claims, **characterised in that** a plurality of identical or different microreactors are connected one after the other in series; especially **characterised in that** at least two (especially at least three) microreactors are connected in series.

15. Process according to any one of the preceding claims, **characterised in that** the reaction between the compound of the formula (II) and nitric acid is carried out in a microreactor as a continuous process and the product from that microreactor is continuously transferred to a further microreactor, in which further microreactor at least one acid anhydride and the chloride-containing catalyst are added.

## Patentansprüche

1. Verfahren zur Herstellung einer Verbindung der Formel (I): 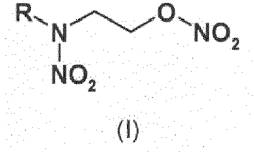
worin R eine Alkylgruppe mit 1 bis 6 Kohlenstoffatomen oder eine Gruppe der Formel -CH₂-CH₂-O-NO₂ ist, oder von Mischungen von zwei oder mehr solcher Verbindungen, das die Umsetzung einer Verbindung der Formel (II): 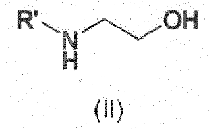
worin R' eine Alkylgruppe mit 1 bis 6 Kohlenstoffatomen oder eine Gruppe der Formel -CH₂-CH₂-OH ist, oder die Umsetzung von entsprechenden Mischungen von zwei oder mehr solcher Verbindungen
mit Salpetersäure und
dann mit einer Mischung von mindestens einem Säureanhydrid und einem chloridhaltigen Katalysator in einem kontinuierlichen Prozess umfasst,
**dadurch gekennzeichnet, dass** die Umsetzungen bei einem Druck von 2 bar über Atmosphärendruck bis zu einem Druck von 80 bar über Atmosphärendruck durchgeführt werden.

2. Verfahren nach Anspruch 1, **dadurch gekennzeichnet, dass** das Reaktionsgemisch nach den Umsetzungen mit Wasser oder einer wässrigen alkalischen Lösung in Kontakt gebracht wird.

3. Verfahren nach Anspruch 1 oder 2, **dadurch gekennzeichnet, dass** R eine Methyl-, Ethyl- oder Butylgruppe oder eine Gruppe der Formel -CH₂-CH₂-O-NO₂ ist und R' eine Methyl-, Ethyl- oder Butylgruppe oder eine Gruppe der Formel -CH₂-CH₂-OH ist.

4. Verfahren nach einem der vorhergehenden Ansprüche, **dadurch gekennzeichnet, dass** die Umsetzungen in einem oder mehreren Durchflussreaktor(en) durchgeführt werden.

5. Verfahren nach einem der vorhergehenden Ansprüche 1 bis 3, **dadurch gekennzeichnet, dass** die Umsetzungen in einem oder mehreren Röhrenreaktor(en) durchgeführt werden.

6. Verfahren nach einem der vorhergehenden Ansprüche 1 bis 3, **dadurch gekennzeichnet, dass** die Umsetzungen in einem oder mehreren Mikroreaktor(en) durchgeführt werden.

7. Verfahren nach einem der vorhergehenden Ansprüche, **dadurch gekennzeichnet, dass** die Umsetzungen bei einem Druck von 2 bar über Atmosphärendruck bis zu einem Druck von 18 bar über Atmosphärendruck durchgeführt werden, vorzugsweise bei einem Druck von 3 bis 12 bar über Atmosphärendruck.

8. Verfahren nach einem der vorhergehenden Ansprüche, **dadurch gekennzeichnet, dass** die Umsetzung mit Salpetersäure bei einer Temperatur von -20°C bis 30°C durchgeführt wird; insbesondere bei einer Temperatur von -10°C bis 10°C oder bei einer Temperatur von -15°C bis -5°C.

9. Verfahren nach einem der vorhergehenden Ansprüche, **dadurch gekennzeichnet, dass** die Umsetzung mit einer Mischung von mindestens einem Säureanhydrid und einem chloridhaltigen Katalysator bei einer Temperatur von 20°C bis 60°C durchgeführt wird; insbesondere bei einer Temperatur von 27°C bis 36°C oder bei einer Temperatur von 25°C bis 35°C.

10. Verfahren nach einem der vorhergehenden Ansprüche, **dadurch gekennzeichnet, dass** Essigsäureanhydrid als Säureanhydrid verwendet wird.

11. Verfahren nach einem der vorhergehenden Ansprüche, **dadurch gekennzeichnet, dass** Chlorwasserstoff als chloridhaltiger Katalysator verwendet wird.

12. Verfahren nach einem der vorhergehenden Ansprüche, **dadurch gekennzeichnet, dass** die bei der Umsetzung verwendete Salpetersäure mit einer Konzentration von 75 bis 96 % in Wasser verwendet wird.

13. Verfahren nach einem der vorhergehenden Ansprüche, **dadurch gekennzeichnet, dass** eine Mischung von Ethyl-NENA (R = Ethyl) und Methyl-NENA (R = Methyl) hergestellt wird.

14. Verfahren nach einem der vorhergehenden Ansprüche, **dadurch gekennzeichnet, dass** eine Vielzahl von identischen oder verschiedenen Mikroreaktoren nacheinander in Reihe geschaltet werden; insbesondere **dadurch gekennzeichnet, dass** mindestens zwei (insbesondere mindestens drei) Mikroreaktoren in Reihe geschaltet werden.

15. Verfahren nach einem der vorhergehenden Ansprüche, **dadurch gekennzeichnet, dass** die Umsetzung zwischen der Verbindung der Formel (II) und Salpetersäure in einem Mikroreaktor als kontinuierlicher Prozess durchgeführt wird und das Produkt aus diesem Mikroreaktor kontinuierlich zu einem weiteren Mikroreaktor überführt wird, in dem mindestens ein Säureanhydrid und der chloridhaltige Katalysator zugesetzt werden.

## Revendications

1. Procédé pour la préparation d'un composé de la formule (I) : 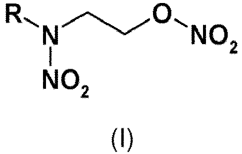
où R est un groupe alkyle ayant de 1 à 6 atomes de carbone ou un groupe de la formule -CH₂-CH₂-O-NO₂, ou un mélange de deux ou plusieurs composés de la sorte,
qui comprend la réaction d'un composé de la formule (II) : 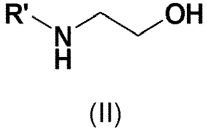
où R' est un groupe alkyle de 1 à 6 atomes de carbone ou un groupe de la formule -CH₂-CH₂-OH, ou la réaction de mélanges correspondants de deux ou plusieurs composés de la sorte,
avec de l'acide nitrique et
ensuite avec un mélange d'au moins un anhydride d'acide et un catalyseur contenant du chlorure dans un processus en continu,
**caractérisé en ce que** les réactions sont exécutées à une pression allant de 2 bar au-dessus de la pression atmosphérique à une pression de 80 bar au-dessus de la pression atmosphérique.

2. Procédé selon la revendication 1, **caractérisé en ce que**, après les réactions, le mélange de réaction est amené en contact avec de l'eau ou une solution alcaline aqueuse.

3. Procédé selon la revendication 1 ou 2, **caractérisé en ce que** R est un groupe méthyle, éthyle ou butyle, ou un groupe de la formule -CH₂-CH₂-O-NO₂, et R' est un groupe méthyle, éthyle ou butyle, ou un groupe de la formule -CH₂-CH₂-OH.

4. Procédé selon l'une quelconque des revendications précédentes, **caractérisé en ce que** les réactions sont exécutées dans un ou plusieurs réacteur(s) à flux continu.

5. Procédé selon l'une quelconque des revendications précédentes 1 à 3, **caractérisé en ce que** les réactions sont exécutées dans un ou plusieurs réacteur(s) tubulaire(s).

6. Procédé selon l'une quelconque des revendications précédentes 1 à 3, **caractérisé en ce que** les réactions sont exécutées dans un ou plusieurs microréacteur(s).

7. Procédé selon l'une quelconque des revendications précédentes, **caractérisé en ce que** les réactions sont exécutées à une pression allant de 2 bar au-dessus de la pression atmosphérique à une pression de 18 bar au-dessus de la pression atmosphérique, de préférence à une pression allant de 3 à 12 bar au-dessus de la pression atmosphérique.

8. Procédé selon l'une quelconque des revendications précédentes, **caractérisé en ce que** la réaction avec l'acide nitrique est exécutée à une température allant de -20 °C à 30 °C ; en particulier à une température allant de -10 °C à 10 °C ou à une température allant de -15 °C à -5 °C.

9. Procédé selon l'une quelconque des revendications précédentes, **caractérisé en ce que** la réaction avec un mélange d'au moins un anhydride d'acide et un catalyseur contenant du chlorure est exécutée à une température allant de 20 °C à 60 °C ; en particulier à une température allant de 27 °C à 36 °C ou à une température allant de 25 °C à 35 °C.

10. Procédé selon l'une quelconque des revendications précédentes, **caractérisé en ce qu'**un anhydride acétique est utilisé comme anhydride d'acide.

11. Procédé selon l'une quelconque des revendications précédentes, **caractérisé en ce qu'**un chlorure d'hydrogène est utilisé comme catalyseur contenant du chlorure.

12. Procédé selon l'une quelconque des revendications précédentes, **caractérisé en ce que** l'acide nitrique utilisé dans la réaction est utilisé avec une concentration dans l'eau allant de 75 à 96 %.

13. Procédé selon l'une quelconque des revendications précédentes, **caractérisé en ce qu'**un mélange d'éthyle-NENA (R = éthyle) et de méthyle-NENA (R = méthyle) est préparé.

14. Procédé selon l'une quelconque des revendications précédentes, **caractérisé en ce qu'**une pluralité de microréacteurs identiques ou différents sont connectés les uns après les autres en série ; en particulier **caractérisé en ce qu'**au moins deux (en particulier au moins trois) microréacteurs sont connectés en série.

15. Procédé selon l'une quelconque des revendications précédentes, **caractérisé en ce que** la réaction entre le composé de la formule (II) et l'acide nitrique est exécutée dans un microréacteur en tant que processus en continu et le produit provenant de ce réacteur est transféré en continu à un autre microréacteur, autre microréacteur dans lequel au moins un anhydride d'acide et le catalyseur contenant du chlorure sont ajoutés.
